# EUROPEAN PATENT SPECIFICATION

(11) **EP 1 932 852 B1**
(45) Date of publication and mention of the grant of the patent: **24.02.2010**
(21) Application number: 06026026.2
(22) Date of filing: 15.12.2006
(51) Int. Cl.: C07K 7/02, C07K 7/06, C07K 14/47, C07K 14/705, A61K 38/08, A61K 38/17

(54) **Novel taxane related peptides and uses thereof**
Neuartige mit Taxan verwandte Peptide und deren Verwendung
Peptides liées au taxane originales et utilisations de celles-ci

(43) Date of publication of application: 18.06.2008
(73) Proprietor: Campiani, Giuseppe, 53042 Chianciano Terme SI (IT); Fattorusso, Caterina, 80128 Napoli (IT); Universita Cattolica Del Sacro Cuore, 00168 Roma (IT)
(72) Inventor: Scambia, Giovanni, 00136 Roma (IT); Ferlini, Cristiano, 00125 Roma (IT); Campiani, Giuseppe, 53042 Chianciano Terme (Siena) (IT); Fattorusso, Caterina, 80128 Napoli (IT)
(74) Representative: Capasso, Olga

(56) References cited:
- EP-A- 1 489 179
- DATABASE REGISTRY XP002431829 retrieved from STN Database accession no. 432071-56-2 & WO 01/81581 A2 (CORIXA CORP [US]; SKEIKY YASIR A W [US]; PERSING DAVID H [US]; MITCHAM) 1 November 2001 (2001-11-01)
- DATABASE REGISTRY XP002431830 retrieved from STN Database accession no. 716760-98-4 & US 2004/123343 A1 (LA ROSA THOMAS J [US] ET AL) 24 June 2004 (2004-06-24)
- LIN BINGZHEN ET AL: "Conversion of Bcl-2 from protector to killer by interaction with nuclear orphan receptor Nur77/TR3" CELL, CELL PRESS, CAMBRIDGE, NA, US, vol. 116, no. 4, 20 February 2004 (2004-02-20), pages 527-540, XP002400324 ISSN: 0092-8674

## Description

The present invention relates to peptides mimicking the pro-apoptotic effects of taxane antitumor agent paclitaxel.

### BACKGROUND OF THE INVENTION

Taxanes represent the most important class of antitumor agents introduced in cancer therapy in the last decade. One member of the family is paclitaxel (PTX), firstly isolated from Taxus Brevifolia and found active as antitumor agent at the end of the 60's. In the mid 90's, a semi-synthetic taxane derived from 10-deacetylbaccatin III was introduced and thereafter named as docetaxel. Taxanes act by inhibiting microtubule dynamics, thereby inducing the arrest in M phase and the consequent activation of the apoptotic program. Paclitaxel and docetaxel are two of the most important anticancer drugs today. However, recent reports have shown that treatment with these drugs often encounters undesirable side effects as well as drug resistance. Therefore, it is important to develop new taxane and taxane-mimicking anticancer agents with fewer side effects, superior pharmacological properties, and improved activity against drug-resistant human cancers.

Paclitaxel binds beta-tubulin and the Bcl-2 protein at the mitochondrial level.

Bcl-2 represents the most important member of the anti-apoptotic factors in the Bcl-family. It is often overexpressed in human cancers and particularly in the follicular B-cell Lymphoma. Bcl-2 acts physiologically as a proton efflux pump preventing conductivity of permeability transition pore channel. Bcl-2 interacts with the protein factor Nur77. This interaction is mediated by the N-terminal loop region of Bcl-2 and is required for apoptosis.

Nur77, an orphan member of the steroid/thyroid/retinoid nuclear receptor superfamily, plays key roles in regulating growth and apoptosis. The mechanism by which Nur77 targets mitochondria and induces apoptosis has been recently investigated by Zhang and coworkers (Cell 2004, 116, 527-540). They discovered that Nur77 translocates from the nucleus to the cytoplasm targeting to mitochondria, inducing cytochrome c release. The "ligand binding domain region" of Nur 77 (377-598) interacts with mitochondrial Bcl-2 protein through the N-terminal loop of this latter and this interaction is able to change the function of Bcl-2 from a protector to a cell killer thus triggering apoptosis.

Bcl-2 is an antiapoptotic protein formed by four conserved domains (BH1, BH2, BH3 and BH4) that are shared with the other members of the Bcl-2 family. Between BH4 and BH3, there is an unstructured region known as "disordered loop". The authors of the present invention provide evidence that paclitaxel is able to target mitochondria by directly interacting with the disordered loop of Bcl-2, thereby stimulating the opening of the permeability transition pore channel and ultimately reverting Bcl-2 function from anti-apoptotic to pro-apoptotic. Moreover, they discovered through computer-assisted molecular modeling that the amino acid composition of the binding site of paclitaxel in the disordered loop of Bcl-2 is similar to that of β-tubulin, thereby suggesting that it represents a common binding domain for a peptide ligand. This prompted the authors to investigate if paclitaxel is a peptidomimetic factor. Since, like paclitaxel, Nur77 reverts the Bcl-2 function from protector to cell killer, they tested the hypothesis that paclitaxel mimics the activity of Nur77. This premise was confirmed since Nur77 is able to interact with both paclitaxel targets, namely disordered loop of Bcl-2 and β-tubulin, and to elicit the same phenotypic changes induced by paclitaxel, namely Bcl-2 phosphorylation at the mitochondrial level and class III beta-tubulin overexpression in the microtubule compartment. Finally, they identified a structural region of Nur77 that includes "the PTX death message," which is able to reproduce the effects of PTX at the mitochondrial and tubulin levels.

Based on these results, the authors designed a peptide motif able to mimic the pro-apoptotic effects of PTX.

### DISCLOSURE OF THE INVENTION

Therefore an object of the invention is a peptide consisting of the amino acid sequence LVPPPPIVGYF or LVPPPPIVPYF.

Another object of the invention is a fused chimeric protein comprising a peptide described above.

A further object of the invention is a conjugated protein comprising a peptide of the invention.

Fusion proteins including amino acid sequences as above described are comprised within the scope of the present invention. Fusion protein may be synthesized by synthetic or recombinant techniques. As example fusion proteins may be obtained to facilitate purification (poly-histidine tag, C-terminal extension, peptide for which a binding agent is available). Other examples include Fc fusion, linker, GST seq etc. The polypeptides of the invention can be in the form of active conjugates or complex with a heterologous moiety, which may be selected from cytotoxic agents, labels (e.g. biotin, fluorescent labels), drugs or other therapeutic agents, covalently bound or not, either directly or through the use of coupling agents or linkers. Useful conjugates or complexes can be generated using molecules and methods known per se in the art, in order to allow therapeutic efficacy (cytotoxic agents, drugs or other therapeutic agents). Cytotoxic agents include chemotherapeutic agents; toxin (e.g., an enzymatically active toxin of bacterial, fungal, plant, or animal origin, or fragments thereof), or a radioactive isotope (i.e., a radioconjugate).

Useful conjugates or complexes can also be generated for improving the agents in terms of drug delivery efficacy. For this purpose, the peptides of the invention can be in the form of active conjugates or complex with molecules such as polyethylene glycol and other natural or synthetic polymers (Harris JM and Chess RB, Nat Rev Drug Discov. (2003), 2(3):214-21; Greenwald RB et al., Adv Drug Deliv Rev. (2003), 55(2):217-50; Pillai O and Panchagnula R, Curr Opin Chem Biol. (2001), 5(4):447-51). In this regard, the present invention contemplates chemically modified peptides as disclosed herein, in which the peptide is linked with a polymer. The polymer may be branched or unbranched. Moreover, a mixture of polymers can be used to produce the conjugates. The conjugates used for therapy can comprise pharmaceutically acceptable water-soluble polymer moieties. Suitable water-soluble polymers include polyethylene glycol (PEG), monomethoxy-PEG, mono- (C1-C10) alkoxy-PEG, aryloxy- PEG, poly- (N-vinyl pyrrolidone) PEG, tresyl monomethoxy PEG, PEG propionaldehyde, bis-succinimidyl carbonate PEG, propylene glycol homopolymers, a polypropyleneoxide/ethylene oxide co-polymer, polyoxyethylated polyols (e.g., glycerol), polyvinyl alcohol, dextran, cellulose, or other carbohydrate-based polymers. Suitable PEG may have a molecular weight from about 600 to about 60,000, including, for example, 5,000, 12,000, 20,000 and 25,000. A conjugate can also comprise a mixture of such water-soluble polymers.

The application of the peptide molecules of the invention is therapy, in particular cancer therapy, more in particular in solid tumors exhibiting sensitivity to paclitaxel. Examples of cancers include but are not limited to advanced ovary, breast, head and neck and lung carcinomas.

It is another object of the invention a pharmaceutical composition comprising a therapeutically effective amount of the peptide and suitable diluents and/or excipients and/or adjuvants.

A therapeutically effective amount depends by the dosage unit form, by the route of administration and by other factors known in the art.

Route of administration include but are not limited to parenteral, subcutaneous, oral, nasal route.

The invention will be described in more detail in the following experimental section by reference to the following figures:

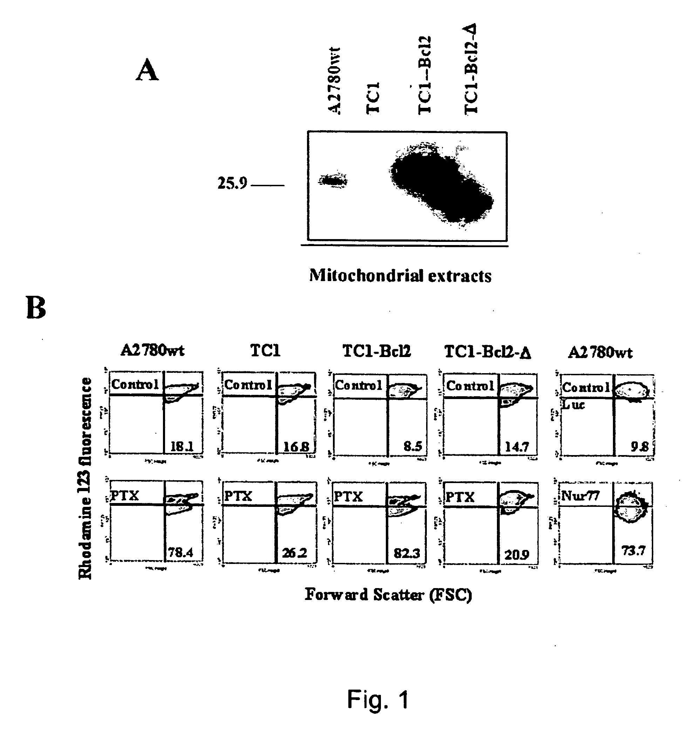

**Fig.1****: A:** Western Blot (polyclonal Ab N-19) showing Bcl-2 expression in mitochondrial extracts (from left to right) in A2780wt, TC1, TC1-Bcl-2, TC-1 Bcl-2Δ. **B:** Analysis of Δψₘ in freshly isolated mitochondria with or without 60 min. of treatment with PTX (10 µM). From left to right A2780wt, TC1, TC1-Bcl-2, TC-1 Bcl-2Δ. In the lower right quadrants the number indicates the percent of mitochondria with opening of PTPC and consequent lekage of Δψₘ. This experiment was repeated three times with similar results.

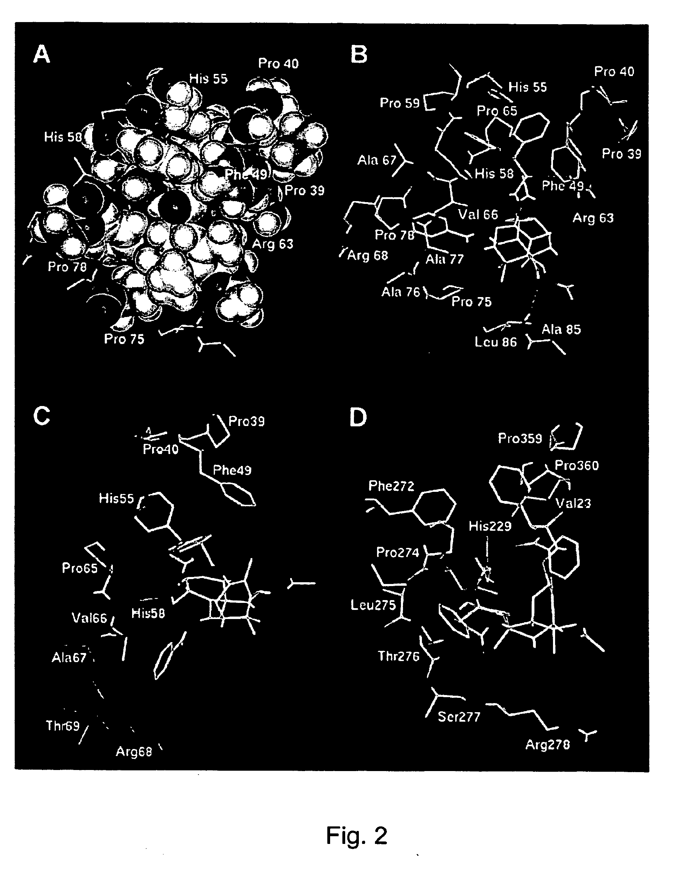

**Fig. 2: (A)** and **(B):** PTX docked into the Bcl-2 loop domain. **(A)** PTX (white) and bcl-2 residues (green) establishing hydrophobic interactions are displayed with their Van der Waals volumes. Heteroatoms are colored by atom type: red for O and blue for N. Hydrogen bonds are highlighted by green dashed lines. Hydrogens are omitted for sake of clarity, with the exception of those involved in hydrophobic interactions (A) and hydrogen bonds; **C** and **D:** Comparison between PTX binding site in Bcl-2 (C) and β-tubulin (D). Proteins motifs involved in these binding sites are evidenced. The carbons of the WW domain ligand, of the polyproline domain and of the LXXLL motif are colored in magenta, in cyan, and in orange, respectively. The carbons of Phe49 are colored in yellow.

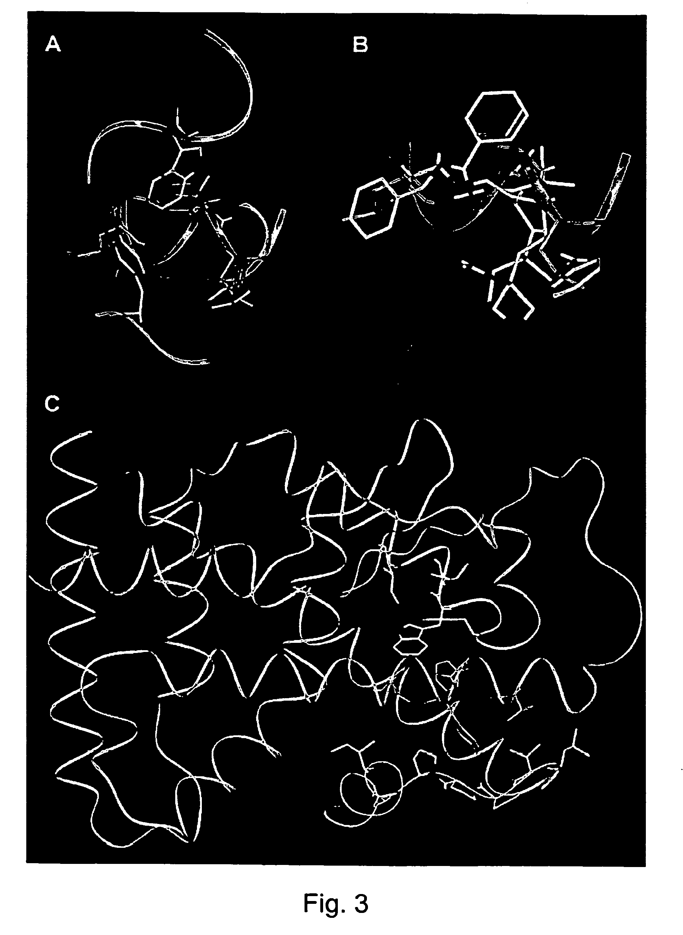

**Fig. 3** **A:** Superimposition of the interacting residues corresponding to the domain WW (carbons in magenta), SH3 (carbons in green) and LXXLL (carbons in orange). The interacting residues are shown as stick. Oxygens are colored in red and nitrogens in blue. Hydrogens are omitted for sake of clarity; **B:** Superimposition between PTX (carbons in white) and LXXLL motif interacting residues (carbons in orange). C: Overall view of the C-terminal of the ligand binding domain of Nur77. Polyproline segment (Leu580-Va1587, carbons in cyan), LXXLL-like motif interacting residues (Va1402- Leu409, carbons in orange; Phe478-Leu486, carbons in yellow) are shown.

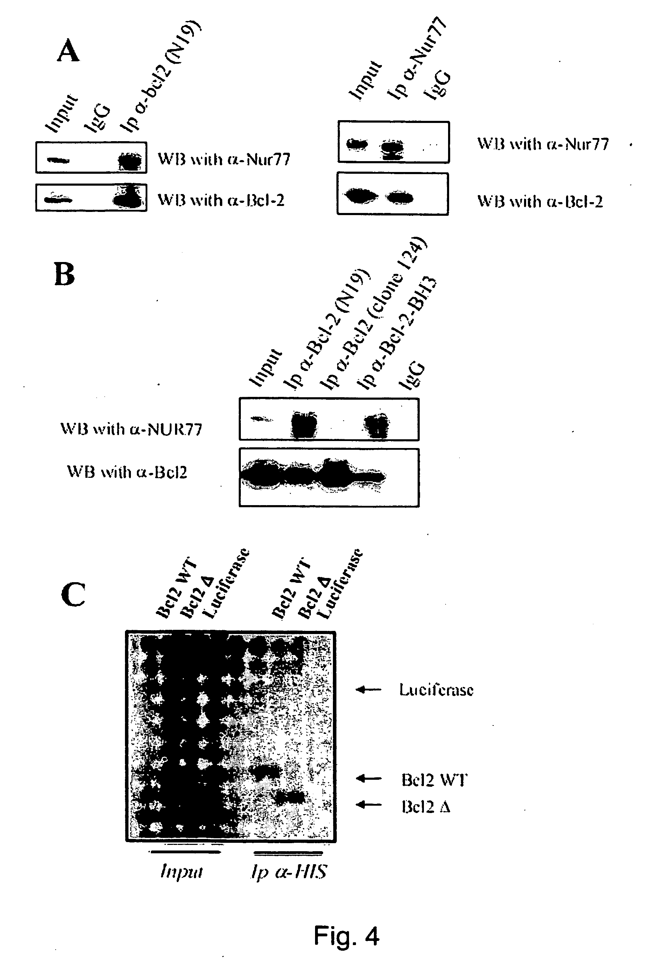

**Fig. 4****: A:** Coimmunoprecipitation of Bcl-2 with Nur77 in whole cell extracts of A2780wt; **B:** Coimmunoprecipitation of Nur77 with Bcl-2 with a panel of anti-Bcl-2 antibodies. From left to right: input, polyclonal N19, loop specific clone 124 and BH-3 specific, control IgG. C: Vitro/Vitro interaction of recombinant Histidine-tagged Nur77 with Bcl-2, and Bcl-2Δ labeled with ³⁵S-methionine. In the left lanes input, in the right columns immunoprecipitation with anti-Histidine antibody. Luciferase was used as internal negative control. Arrows indicate the specific bands for luciferase, Bcl-2wt and Bcl-2Δ.

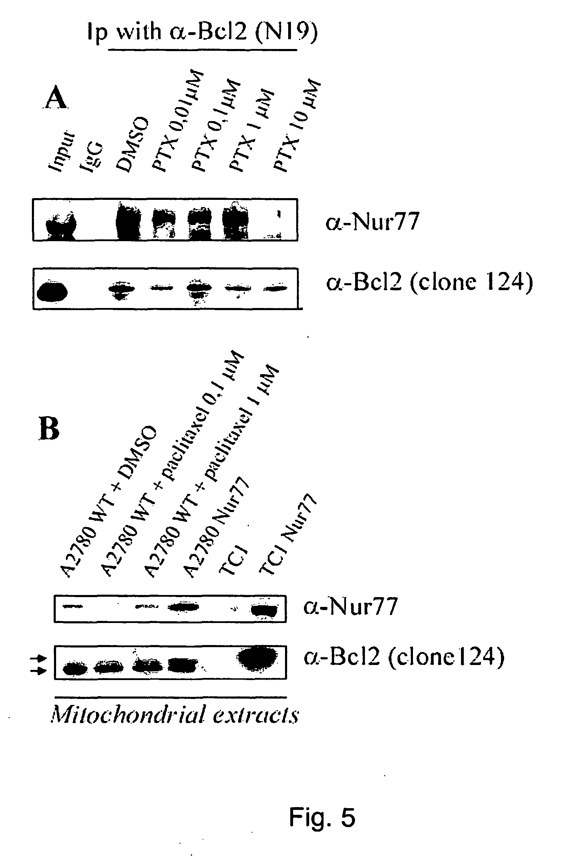

**Fig. 5****: A:** Coimmunoprecipitation of Nur77 with Bcl-2 in the presence of increasing concentration of PTX. From left to right: Input, negative control, vehicle (DMSO 0.1%), PTX 0.01, PTX 0.1, PTX 1 and PTX 10 µM. **B:** Western blot analysis of Nur77 and Bcl-2 in mitochondrial extracts (from left to right) of A2780wt+DMSO 0.1%, A2780wt treated 24h with PTX 0.1 µM, A2780wt treated 24h with PTX 1 µM, A2780Nur77, TC1 and TC1Nur77. The slow mobility band was detectable in A2780wt treated with PTX 1 µM, A2780Nur77 and TC1Nur77.

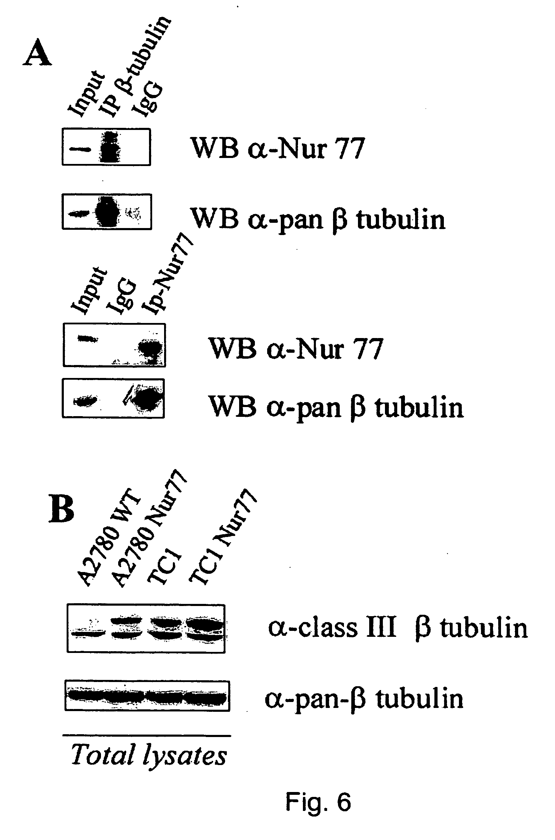

**Fig. 6****: A:** Coimmunoprecipitation in whole cell lysates of A2780wt of Nur77 with β-tubulin. **B:** Western blot analysis of class III β-tubulin (from left to right) in A2780Nur77, A2780wt, TC1 and TC1 Nur77. Overexpression of class III β-tubulin and its post-transcriptional modification was detectable in TC1 cells (chronically exposed to PTX to maintain the resistant phenotype) as well as in A2780Nur77.

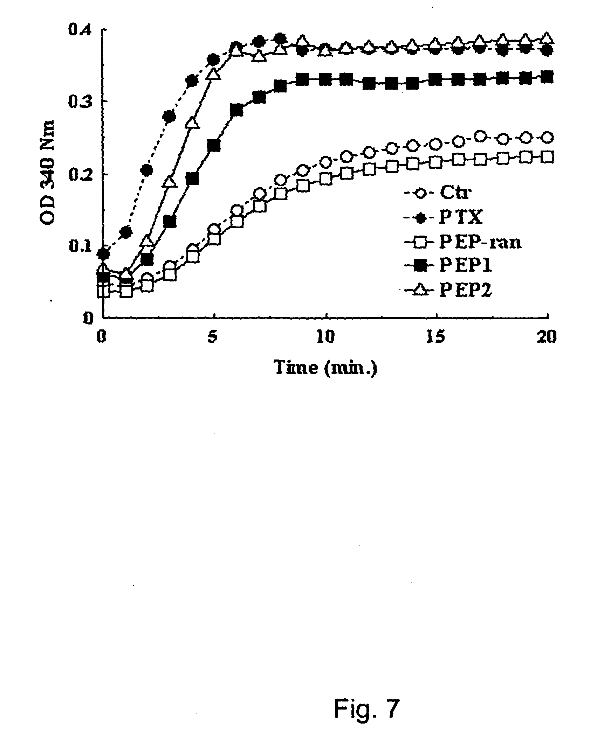

**Fig. 7****:** Line chart showing tubulin polymerization experiments with the control (open circle, dashed line), PTX (closed circle, dashed line), PEP1 (closed square), PEP2 (open triangle) and PEP-ran (open square). All the compounds were used at 10 µM. Experiments were carried out four times with similar results.

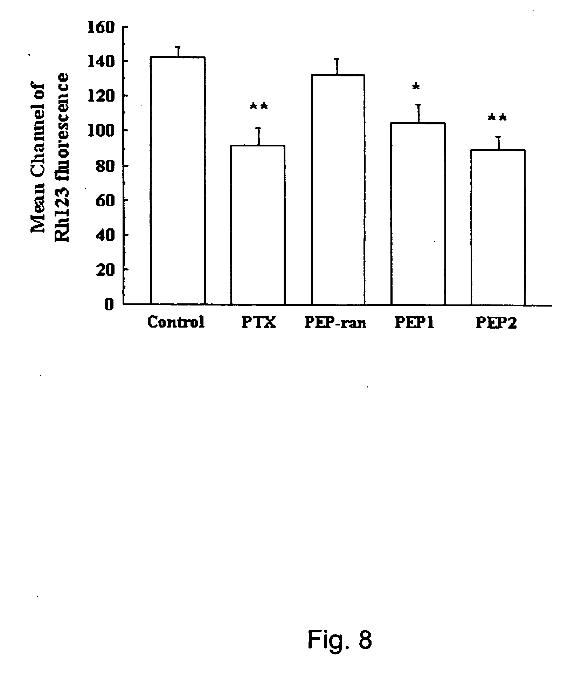

**Fig. 8**: Bar chart resuming experiments on flow cytometric assessment of ΔΨₘ using the three peptides and PTX as a positive control. Bars and error bars represent the mean and SD of triplicate samples. Single and double asterisks marks the statistical significance at a level of 0.05 and 0.01, respectively. Used statistical test was Paired T-test. This experiment has been repeated three times with similar results.

### MATERIALS AND METHODS

### Flow cytometry and assessment of Δψₘ

Cell culture procedures and cell lines have been elsewhere described (Ferlini et al., 2003). Mitochondria were freshly isolated and mitochondrial transmembrane potential Δψₘ was assessed as previously described (Ferlini et al., 1995), using Rhodamine 123 (Molecular Probes) and a Facscan flow cytometer (Becton Dickinson).

### Western blot and immunoprecipitation experiments

Cells, harvested in cold phosphate-buffered saline, were extracted for 30 min at 4 °C in lysis buffer (50 mM Tris-HCl, pH 7.4, 150 mM NaCl, 1% NP-40) containing 1mM DTT, protease inhibitor cocktail (Roche) and phosphatase inhibitors (50 mM NaF, 0.2 mM Na3VO4). Mitochondria isolation was performed as previously described (Ferlini et al., 2003). After centrifugation, supernatants (300 µg from total lysates and 100 µg from mitochondrial extracts) were immunoprecipitated at 4 °C overnight in lysis buffer by adding protein A/G plus-agarose beads (Santa Cruz Biotechnology) after 2 h of incubation with 2 µg of the following antibodies: anti-Bcl-2 (N19, Santa Cruz Biotechnology), anti-Bcl-2 (clone 124, Dako), anti-Bcl-2 BH3 domain (Abgent), anti-Nur77 (Imgenex), anti-β-tubulin (H235, Santa Cruz Biotechnology), anti-class III β-tubulin polyclonal antibody (Covance) and isotype-matched non-immune IgG (Sigma) as control. Immunoprecipitation experiments in the presence of PTX were performed for 2 h at 4 °C. Western blots were done as previously described (Ferlini et al., 2003).

### Protein production and in vitro binding experiments

6xHis-tagged Nur77 (Gene ID 3164) was expressed in Escherichia coli strain M15 (pREP4). Expression cultures were grown at 37°C to mid-log phase and induced for 3 h by adding isopropyl-β-d-thiogalactoside (Sigma) to a final concentration of 1mM. Cells were harvested by centrifugation, resuspended in lysis buffer (50mM Na₂HPO4, 300 mM NaCl, 10 mM imidazole, pH 8.0) containing protease inhibitor cocktail (Roche), incubated for 30 min on ice with lysozyme (1mg/ml) and sonicated. 6xHis-tagged Nur77 was purified using Ni-NTA agarose (Qiagen) according to the manufacturer's protocols.

In vitro translated proteins were produced using a coupled reticulocyte transcription-translation system according to the manufacturer's protocol (Promega). For in vitro binding experiments, 2 µg of purified 6xHis-tagged Nur77 was incubated with in vitro translated ³⁵S-methionine-labeled proteins in binding buffer (50 mM Tris-HCl, pH 7.4, 300 mM KCl, 20 mM imidazole) containing protease inhibitor cocktail (Roche) and phosphatase inhibitors (50 mM NaF, 0.2 mM Na₃VO₄) on a rotating wheel for 90 min at 4 °C in the presence of 10 µl of Ni-NTA Magnetic Agarose Beads (Qiagen). The beads were immobilized on a magnetic separator, the supernatant removed, and following extensive washing with the same buffer, bound proteins were eluted by boiling in SDS-PAGE sample buffer.

### DNA constructs and antibodies

Human full-length Bcl2 cDNA was obtained (Gene id:596; Accession number NM_000633) from lymphocytes by reverse transcription-PCR with Pfu DNA polymerase (Promega), using the following primers: forward 5'-TTAAGCTTATGGCGCACGCTGGGAGAACAGGGT-3' and reverse 5'-CCTCTAGATTCACTTGTGGCCCAGATAGGCACC-3'and HindIII/XbaI-cloned into pUSE (Upstate Biotechnology).

A construct (pUSE-Bcl2Δ) was made in which 49 aa (32-80) were deleted by reverse PCR and replaced with a linker of 4 alanines.

Generation of full-length human Nur77 cDNA (NM_173158) was performed by PCR from human testis cDNA library (ResGen) with Pfu DNA polymerase (Promega), using the following primers:
forward 5'-TTACAGGATCCAGATGCCCTGTATCCAAGC-3' and reverse 5'-GCCCGAATTCGGTCAGAAGGGCAGCGTGTC-3' The PCR product was cloned into the BamH1 and EcoRI site of pUSE. The PCR product was then isolated after the BamH1 and EcoRI digestion and subsequently cloned into pQE1 (Qiagen). All PCR products were sequenced by 3100-Avant Genetic Analyzer (Applied Biosystem)

The antibodies used were: anti-class III β-tubulin polyclonal antibody (Covance, Berkeley, CA), anti-β-tubulin (H-235, Santa Cruz Biotechnology), anti-Bcl2 (clone 124, Dako), anti-Bcl2 (N19, Santa Cruz Biotechnology), anti-Bcl2 BH3 domain (Abgent) and anti-Nur77 (Imgenex).

### Modeling of Bcl-2

All molecular modeling studies were performed on SGI Indigo II R10000 and SGI Octane 2XR10000 workstations. The NMR structures of Bcl-2 (PDB IDs: 1GJH, 1G5M) and the experimentally determined structures of Bcl-xL (PDB IDs: 1MAZ, 1LXL, 1R2D, 1G5J, 1PQ0, 1BXL, 1PQ1, 1YSG, 1YSI, 1YSN) were downloaded from the Protein Data Bank (PDB; http://www.rcsb.org/pdb/). Hydrogens were added to all the PDB structures considering a pH value of 7.2. Since both Bcl-2 NMR structures lack the loop region (amino acids 35-91), the human Bcl-2 homology model was built using the standard homology building procedure provided by the Homology module of Insight 2005 (Accelrys). PTX structure was unmerged (Biopolymer Module, Insight 2005) from the structure of the complex with α-β tubulin (http://www.rcsb.org/pdb/; PDB IDs: 1JFF). PTX was placed in the disordered loop region of the protein using the merge command in the Biopolymer Module. Obtained complex was subjected to preliminary energy minimization to generate roughly docked starting structure, as required by Affinity docking procedure. The minimization procedure was performed as follows: Steepest Descent algorithm was used until the maximum RMS derivative was less than 5.0 kcal/Å then Conjugate Gradient algorithm was used until the maximum RMS derivative was less than 0.001 kcal/Å. The dielectric constant was set at 80*r to mimic the aqueous environment of the cytoplasm. During the minimization, the whole disordered loop backbone and sidechains (amino acids 35-91) were left free to move. Flexible docking was achieved using the Affinity module in the Insight 2005 suite, setting the SA_Docking procedure, and using the Cell_Multipole method for non-bond interactions. The binding domain area was defined as a subset including the ligand and all the residues of the unstructured loop (amino acids 35-91). All atoms included in the binding domain area, were left free to move during the entire docking calculations. A Monte Carlo/minimization approach for random generation of a maximum of twenty structures was used, with an energy tolerance of 10⁶ Kcal/Mol, to ensure a wide variance of the input structures to be minimized (2500 iterations; ε=80*r). The Metropolis test, at a temperature of 310 K, and a structure similarity check (RMS tolerance = 0.3 kcal/Å), were applied to select acceptable structures. 50 stages of Simulated Annealing (100 fs each) were applied on the resulting complexes. Over the course of the Simulated Annealing, system temperature was linearly decreased from 500 K to 300 K, concurrently the Van der Waals and Coulombic scale factors were similarly decreased from their initial values (defined above as 0.1) to their final value (1.0). A final round of 10⁴ minimization steps was performed at the end of the molecular dynamics. After this procedure, the resulting docked structures were ranked by their conformational energy. Ligand-enzyme interaction energy of each complex has been evaluated by calculating the nonbond interaction energy between the ligand and each protein residues within the binding domain area, using the Discover_3 Module of Insight 2005. The complex with the best compromise between the total energy and the binding energy was selected as structure representing the most probable binding mode.

The peptides LVPPPPIVGYF and LVPPPPIVPYF were built in an extended form using the Residue/Append command (Biopolymer Module, Insight 2005). The N-terminal and the C-terminal were considered un-charged.

Conformational space of the peptides was sampled through 200 cycles of Simulated Annealing (CVFF force field, ε= 80*r) by following this protocol: an initial temperature of 1000 K was applied to the system for 2000 fs with the aim of surmounting torsional barriers; successively temperature was linearly reduced to 300 K in 1000 fs. Resulting structures were subjected to energy minimization within Insight 2005 Discover module (CVFF force field, Conjugate Gradient algorithm; ε=80*r) until the maximum RMS derivative was less than 0.01 kcal/Å and subsequently ranked by their conformational energy and torsional angle values.

### Bioinformatics analysis

The linear functional motifs present in β-tubulin and Bcl-2 PTX binding sites were identified by using the Eukaryotic Linear Motif server http://elm.eu.org/, a resource for predicting small functional sites in eukaryotic proteins, such as those subject to post-translational modification or involved in protein-protein interactions (Puntervoll et al., 2003).

The experimentally determined structure of: SH3 domains (PDB IDs: 1QWE, 1 QWF), LxxLL motifs (PDB IDs: 1SB0, 1T7F), WW domain (PDB IDs: 1EG4), the orphan nuclear receptor Nur77 ligand-binding domain (PDB IDs: 1 YJE), and the α-β tubulin dimer in complex with PTX (PDB IDs: 1JFF), were downloaded from the Protein Data Bank (PDB; http://www.rcsb.org/pdb/). Hydrogens were added to all the PDB structures considering a pH value of 7.2 (Biopolymer Module, Insight 2005).

Sequence alignments were performed using ClustalW (WWW Service at the European Bioinformatics Institute, http://www.ebi.ac.uk/clustalw) and Multiple_Alignment (Accelrys, San Diego). Results were analyzed using the Insight 2005 Homology Module (Accelrys, San Diego). The calculation of the protein backbone dihedral angles (phi and psi) was performed on the PDB structures using the Residue_Dihedral command (Homology Module, Insight 2005).

The centroids of the interacting residues were determined taking in account the heavy atoms of the amino acids side chains and the heavy atoms of PTX phenyl rings (Pseudo_Atom Define command, Biopolymer Module, Insight 2005).

### Cell free tubulin polymerisation assays

Purified Bovine brain tubulin was purchased by Cytoskeleton (Denver, CO). The ability of the compounds to enhance tubulin polymerization was determined as previously described 14. Briefly, tubulin (1.0 mg/ml) was polymerized in PEM buffer (80mM Pipes pH6.9, 2mM MgCl2, 0.5mM EGTA, 5% glycerol, 1mM GTP) in the presence of a range of drug concentrations in a Beckman spectrophotometer equipped with a constant-temperature cuvet chamber. Polymerization was quantified by the measurement of turbidity at 350 nm.

### RESULTS

The authors have recently demonstrated that Bcl-2 is dramatically downregulated in PTX-resistant cells and that re-expression of the protein partially restores drug sensitivity (Ferlini et al., 2003). Here, they used the human ovary A2780 cancer cell line and its counterpart TC1 (Ferlini et al., 2003) with acquired PTX-resistance. These cells that exhibit an high degree of Bcl-2 downregulation, were then stably transfected with a construct containing the full-length human BCL2 gene (TC1-Bcl-2 cells). The phenomenon of Bcl-2 downregulation is specific for Bcl-2, and does not involve the other important anti-apoptotic factor Bcl-xL (Ferlini et al., 2003). Since the homology between the Bcl-2 and Bcl-xL is high, with the exception of the loop domain, TC1 cells were stably transfected with a construct in which the loop domain of Bcl-2 (aa 35-91) was deleted and replaced with a linker sequence of four alanine residues, as previously described by Chang et al. (Chang et al., 1997). These cells are thereafter referred to as TC1 Bcl-2Δ. The expression levels of Bcl-2 for the cells used in this study are shown in Fig. 1A. Mitochondria were freshly isolated from these cells and were treated with PTX (10 µM). This concentration has a clinical relevance since it corresponds to the intracellular concentration achievable in patients. (Andre et al., 2000).

The status of mitochondrial transmembrane potential (Δψₘ) was then monitored using the cationic fluorochrome Rhodamine 123 and flow cytometry. Results of these experiments are depicted in Fig. 1B. In A2780wt cells, PTX treatment is able to induce a massive opening of the Permeabilty Transition Pore Channel (PTPC), as demonstrated by the dissipation of the Δψₘ occurring in the vast majority of mitochondria after 60 min. after addition of PTX. In fact, as reported in Fig. 1B the percentage of mitochondria with full leakage of the Δψₘ pass from 18% to 78%. On the other hand, in TC1 cells with undetectable levels of Bcl-2, PTX treatment only marginally increased the number of mitochondria with the leakage of Δψₘ (from 16% to 26%). In TC1-Bcl-2 cells, the sensitivity to PTX treatment was restored and again the vast majority of treated mitochondria were able to open the PTPC upon PTX treatment and dissipate the Δψₘ (from 8% to 82%). Finally, in TC1-Bcl-2Δ, the sensitivity to PTX was abolished and mitochondria did not relevantly change the Δψₘ status upon PTX treatment (from 14% to 20%). Taken together, these findings indicate that PTX is able to convert Bcl-2 in a factor proficient to activate the opening of PTPC and that such activity requires the presence of Bcl-2 loop domain, since it is not longer detectable in mitochondria from TC1-Bcl-2Δ cells.

### Molecular modeling

Since previous work has suggested that PTX directly interacts with Bcl-2 (Rodi et al., 1999) at the level of disordered loop (Wu et al., 2000), PTX has been docked in to the homology model of the Bcl-2 loop domain by using a mean of Monte Carlo/minimization and Simulated Annealing techniques; during the entire calculation procedure either PTX or Bcl-2 disordered loop (amino acids 35-91) were left fully free to move. Twenty different structures have been generated and the final complex was selected on the basis of both its potential and binding energies. The resulting binding site is reported in Fig. 2A, B. Bcl-2 loop rearranged on PTX structure thus forming an hydrophobic environment in which PTX skeleton is embedded and which includes several aromatic residues (Phe49, His55, His58) establishing π-π interactions with the aromatic rings of the ligand, and many aliphatic residues such as Pro65, Val66, Pro75, Ala77, Pro78, as depicted in Fig. 2A. On the other hand, the side chains of Arg63 and the amide hydrogen of Leu86 are involved in hydrogen bonding to donor key groups of PTX. The whole Bcl-2 PTX binding site showed striking similarities with that of β-tubulin (PDB code: 1JFF)

(Lowe et al., 2001), conserved amino acids including P65-R68 stretch (P274-R278 in β-tubulin), P39-P40 (P359-P360 in β-tubulin), F49 (F272 in β-tubulin), H55 (V23 in β-tubulin), and H58 (H229 in β-tubulin) (Fig. 2C, D). Interestingly, both Bcl-2 and β-tubulin PTX binding sites contains a poly-proline cleft together with several hydrophobic/aromatic residues. Poly-proline containing sequences were also found in a library of phage-displayed peptides able to bind PTX (Rodi et al., 1999) prompting us to investigate a possible role of PTX as peptide-mimetic, capable to interfere with protein-protein interactions involving poly-proline ligands. In support of this hypothesis, the Eukaryotic Linear Motifs software (ELM; Puntervoll et al., 2003) identified two SH3 domain ligands (72-78 and 85-91) and four WW domain ligands (53-58, 67-72, 71-76, and 84-89) in the human Bcl-2 loop (P10415 numbering); indeed, it is known that WW domains and SH3 domains can potentially bind overlapping sites, such as proline-rich peptides. Analogously, a structural investigation of the PTX binding site on β-tubulin (PDB: 1JFF) evidenced the presence of two proline rich domains (359-371 and 272-278, 1JFF numbering) and two LXXLL domain (20-28 and 227-231, 1JFF numbering). The authors therefore investigated the molecular interactions between SH3, WW, and LXXLL domains and their ligands by analyzing several X-ray structures. The main structural features of the interacting residues are reported in Table 1.

**Table 1 : Structural features of the interacting residues of SH3 domain, LxxLL motif and WW domain**

| **SH3 domain ^{a}** | |
|---|---|
| Aminoacids | Distances between side chain centroids |
| Y16 - W42 | 6.19 Å |
| Y60 - Y16 | 6.61 Å |
| Y60 - W42 | 8.38 Å |

| **LxxLL motif^{b}** | |
|---|---|
| Aminoacids | Distances between side chain centroids |
| L95 - L98 | 7.38 Å |
| L99 - L95^{c} | 10.08 Å |
| L99 - L98 | 8.18 Å |

| **WW domain^{d}** | |
|---|---|
| Aminoacids | Distances between side chain centroids |
| T81 - Y72 | 4.51 Å |
| W83 - T81^{e} | 7.76 Å |
| W83 - Y72 | 6.98 Å |

| | |
|---|---|
| ^{a} Structural parameters taken from SH3 domain X-ray structure (PDB IDs: 1QWE, 1QWF) ^{b} Structural parameters taken from LxxLL domain X-ray structure (PDB IDs: 1SB0, 1T7F) ^{c} L95 and L99 are residues i and i+4 in an α-helix (PDB IDs: 1SB0, 1T7F) ^{d} Structural parameters taken from WW domain X-ray structure (PDB ID: 1EG4) ^{e} T81 and W83 are residues i and i+2 in a β-strand (PDB ID: 1EG4) | |

According to their ligand selectivity, SH3 and WW domains share common structural features: interestingly, these structural features are also similar to those displayed by the LXXLL motif, in which the first and the last Leu residues (i, i+4; alpha helix) are structured like the interacting residues Thr and Trp i+2 (i, i+2; β-strand) in the WW domain (Table 1, Fig. 3A), in agreement with a recent report (Fasan et al., 2004) that the distance between C_{α} atoms of residues i and i+4 on one face of an alpha helix is similar to the distance between C_{α} atoms of residues i and i+2 along one strand of a β-hairpin.

The authors also calculated the structural features of paclitaxel extracted from the X-ray structure 1JFF (PDB code); the results are reported in Table 2.

**Table 2: Structural features of Paclitaxel**

| | | | |
|---|---|---|---|
| 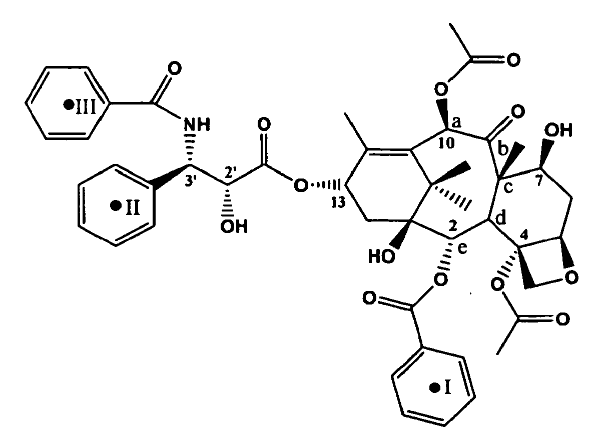 | | | |

| Distances between centroids | | Torsional angle | |
|---|---|---|---|
| Rings | Values | Atoms | Values (°) |
| | (Å) | | |
| II-III | 6.96 | τ_{abcd} | -50.68 |
| I-II | 9.60 | τ_{bcde} | -52.94 |
| I-III | 8.28 | | |

There is a striking similarity between the distances among the PTX "side chains" (I, II, and III in Table 2) and the distances among the side chains of interacting residues in the SH3, LXXLL, and WW domains. Moreover, the PTX structure includes a turn-like structure around rotatable bonds C3-C8 and C8-C9 that is compatible with both a type I-α_{RS} alpha turn and a type I-III β turn (Table 2, Fig. 3B). Overall the PTX skeleton resembles an alpha helix structure in which rings II, III, and I represents the side chains of residues i, i+3, and i+4, respectively (Fig. 3B), thus mimicking an LXXLL-like protein domain. Accordingly, the three aromatic moieties of PTX are engaged in hydrophobic contacts with other similarly oriented hydrophobic residues, both in the β-tubulin binding site (His229, Val23, and Phe272; Fig. 2D) and in the author's Bcl-2 docked structure (His58, His55, and Phe49; Fig. 2C), resembling LXXLL domain interactions. On the other hand, the oxetane ring of PTX mimics the shape of a proline residue, and it is engaged in favorable hydrophobic interactions both in β-tubulin (Pro274 and Leu275) and Bcl-2 (Pro65 and Val66) binding sites, similarly to that observed for SH3 domain ligands. Finally, the authors performed a structural analysis on the C-terminal fragment of the experimentally determined structure of the ligand binding domain of Nur77 (PDB: 1YJE, Fig. 3C), reported to strongly interact with Bcl-2 (Lin et al., 2004). This region includes a poly-proline segment (582-585, 1YJE numbering) interacting with three hydrophobic residues (V402, F405, Y406), its structural comparison with SH3 domains in complex with poly-proline ligands (PDB: 1QWE, 1QWF) revealed the same orientation of the interacting residues (Fig. 3). Moreover, residues F405 and Y406 are located in the H3 helix of Nur77 within a sequence containing a repeated LXXLL-like motif (V402-L409, 1YJE numbering). In particular, F405 is also involved in π-π interactions with residues F478 and W481, which are part of another LXXLL-like structure (helix H7, F478-I482, 1 YJE numbering) in the hydrophobic core of the binding domain. Thus, the interaction of Nur77 with Bcl-2 loop could be mediated through an LXXLL-like domain, which could be, in turn, mimicked by PTX, as evidenced by the molecular superimposition reported in Figure 3B. In this view, in order to disclose the Bcl-2 interacting site on Nur77, a conformational change of the C-terminal poly-proline helix would be required.

On the basis of these results, the authors hypothesized that PTX pro-apoptotic activity is due to its ability to mimic a peptide structure containing both an "LxxLL/SH3/WW"-like domain and a poly-proline domain. Accordingly, following this rational approach, they designed the peptides LVPPPPIVGYF (PEP 1), and LVPPPPIVPYF (PEP 2), predicted to display the biological activity of PTX. The activity of the designed peptides proved the authors hypothesis that PTX is a peptidomimetic compound bearing a death signal involved in the apoptotic pathway.

### Peptide-mimicry

The significant similarity between the binding sites of the two PTX target proteins resulted from molecular modeling studies prompted the authors to investigate the possibility that PTX is a peptidomimetic compound. In a previous study it has been reported that Nur77/TR3 interacts with Bcl-2 thereby reverting its function from anti-apoptotic to pro-apoptotic (Lin et al., 2004). This activity resembles PTX activity on isolated mitochondria described above. By means of immunoprecipitation experiments, the authors found that Bcl-2 binds to Nur77 in A2780 cell line (Fig. 4A). In order to better characterize this interaction, a panel of anti-Bcl-2 antibodies was used to perform immunoprecipitation assays using mitochondrial extracts. Both anti-Bcl-2 antibodies directed toward N-terminus (N-19) and the BH3 domain were able to immunoprecipitate the Bcl2/Nur77 complex, whereas the antibody with epitope within the disordered loop (clone 124) did not (Fig. 4B). This result suggests that the Bcl-2 disordered loop is responsible for Nur77 binding and the complex Bcl-2/Nur77 is able to mask the epitope recognized by clone 124 antibody. In order to assess this hypothesis, recombinant histidine-tagged Nur77 was purified and the interaction with in vitro produced Bcl-2 and Bcl-2Δ labeled with ³⁵S-methionine was analyzed. The authors' results show that Nur77 is able to interact with Bcl-2, but not with Bcl-2Δ, confirming that the interaction Bcl2/Nur77 requires the disordered loop (Fig. 4C). To assess if PTX was able to interfere with the Bcl2/Nur77 complex, whole cell lysates from A2780 were treated with increasing concentrations of PTX up to 10 µM and co-immunoprecipitation experiments were performed. As shown in Fig. 5A, at the concentration of 10 µM, PTX was able to displace Nur77 from Bcl-2, thereby suggesting that both PTX and Nur77 target the same site on Bcl-2.

It has already been shown that Bcl-2 is phosphorylated following exposure to PTX and that phosphorylation occurs in the disordered loop of Bcl-2 (Haldar et al., 1996; Fang et al., 1998). In order to investigate if Nur77, like PTX, is able to induce Bcl-2 mobility shifts, A2780 and TC1 cell lines overexpressing Nur77 (thereafter referred to as A2780Nur77 and TC1Nur77) were established and Bcl-2 status was analysed. The classical slow mobility form of Bcl-2 observed after PTX treatment was induced in both A2780Nur77 and TC1Nur77 cell lines (Fig. 5B). Altogether these findings are consistent with a Nur77-induced post-transcriptional modification and/or stabilization of Bcl-2 protein, resembling PTX treatment.

Since the major target of PTX is β-tubulin, we wanted to verify if Nur77 was able to interact with this protein. Our results shows that Nur77 co-immunoprecipitates with β-tubulin (Fig. 6A), thereby demonstrating for the first time that Nur77 functionally interact with β-tubulin. Cells treated with PTX exhibit upregulation of class III β-tubulin (Burkhart et al., 2001). In order to assess if Nur77 is able to induce the same phenotypic effects as PTX at the microtubule level, the authors assessed class III beta-tubulin expression in A2780wt, TC1 and the counterparts stably transfected with Nur77. A2780Nur77 cells exhibit an up-regulation of class III β-tubulin (TUBB3) as compared to not transfected cells and the same mobility shift observed in PTX resistant cell lines. In TC1 cells, made resistant to PTX upon chronic exposure to this drug, class III β-tubulin was overexpressed and no further overexpression was detected in TC1Nur77.

Real time PCR analysis confirmed that this up-regulation occurs at the transcriptional level and the extent is similar in A2780Nur77 and TC1 cells.

In summary, Nur77 stable transfection induced an over-expression of class III β-tubulin in A2780wt, similarly to what observed when these cells were chronically exposed to paclitaxel (becoming resistant to the drug: TC1 cells). In addition, stable transfection of Nur77 in TC1 cells does not induce any variation on the over-expression of class III β-tubulin induced by the chronic exposure to paclitaxel. These results indicate that Nur77 is able to induce the same phenotypic effects as PTX also at the microtubule level.

As described above the authors designed two peptides able to fulfill the structural requirements of paclitaxel mimicry, named PEP1 (LVPPPPIVGYF) and PEP2 (LVPPPPIVPYF). These peptides were designed on the basis of: i) PTX structure and its binding sites on β-tubulin and Bcl-2 ; ii) X-ray structures of SH3, WW, and LXXLL domains in complex with their ligands; iii) the region including the poly-proline segment of Nur77 (L580-V588, 1YJE numbering) and iv) the regions containing a repeated LXXLL-like motif of Nur77 (V402-L409 and F478-I482, 1YJE numbering).

As a control the authors designed a peptide having the common components of PEP1 and PEP2, but in a random sequence (PLIPVYPFVP: PEP-ran). The activity of these peptides was assessed in experiments of tubulin polymerization and flow cytometric measurement of Δψₘ. In tubulin polymerization experiments both PEP1 and PEP2 were able to induce microtubule polymerization (Fig. 7). Using equimolar concentration, PEP2 induced an effect superimposable to that of PTX, whereas PEP1 an effect slightly lower. PEP-ran did not change the kinetics of microtubule polymerization. This finding reveals that a small peptidic structure is able to stimulate microtubule polymerization with the same efficiency as PTX. Being PTX mimicked by a small peptide, the results confirm the peptidomimetic nature of PTX. In order to investigate PTX mimicry of the peptides at the mitochondrial level, flow cytometric measurement of Δψₘ was performed using the three peptides PEP1, PEP2 and PEP-ran and PTX as a positive control. Again, paclitaxel mimicry was observed at the mitochondrial level. PEP2 produced the leakage of Δψₘ with an extent, at equimolar concentration (10 µM), similar to that of PTX and PEP1 an effect slightly lower. As expected, PEP-ran was unable to interfere with Δψₘ (Figure 8). Taken together these findings reveal that PTX biologic activities can be mimicked by a peptide, specifically designed to contain the structural motifs of PTX.

### DISCUSSION

Despite the fact that taxanes are largely employed as anticancer agents, it is still unclear the exact mechanism(s) connecting the disruption of microtubule network to the activation of cell death. In keeping with previous reports (Evtodienko et al., 1996; Andre et al., 2000) the authors provide direct evidence that PTX is able to revert the Bcl-2 function, through a direct targeting on mitochondria. Bcl-2 acts as a gatekeeper of the PTPC (Voehringer, 1999), but, in the presence of PTX, the function of stopper of PTPC is abolished and, conversely, Bcl-2 facilitates the opening of PTPC, with the consequent leakage of Δψₘ and release of the mitochondrial factors able to activate cell death, such as cytochrome c. This phenomenon is likely to underlie the paradox of PTX-induced Bcl-2 downregulation observed in PTX-resistant tumor cells and cancer patients (Ferlini et al., 2003). Molecular modeling helped us to characterize the putative binding site and the interaction involved at the molecular level. The homology between the experimentally defined PTX binding site of beta-tubulin (Lowe et al., 2001), with the site predicted in Bcl-2 is impressive. But why are there at least two PTX protein targets in the cells? Due to the extreme similarities between the binding sites of PTX to Bcl-2 and tubulin, it is conceivable that PTX binding site represents an interaction domain for one or more endogenous peptides and that, consequently, PTX is a compound able to mimic this (these) endogenous peptide ligand(s). This hypothesis was previously suggested by Leu et al (Leu et al., 1994), who discovered that a monoclonal anti-idiotypic antibody generated against PTX was able to simulate "in vitro" PTX activity, thereby demonstrating that a peptide structure could vicariate the biological activity of PTX. From a functional point of view, the mimicked motif is able to deliver a potent death signal from microtubules to mitochondria. This explains also why throughout the evolution many species have discovered such a motif and used it to increase its-own arsenal of defensive strategies. In fact, albeit not chemically related, several natural compounds such as epothilones (from myxobacterium *Sorangium cellulosum*) and eleutherobins (from a marine soft coral), similarly interact with the PTX binding site on tubulin and share a common pharmacophore (Ojima et al., 1999). Recently, also the protein product of HIV tat has been identified as a ligand for the PTX-binding site in tubulin, and this interaction seems to play a pivotal role in inducing apoptosis of HIV infected T cells (Chen et al., 2002; de Mareuil et al., 2005). But which is the mimicked protein? Given the similarity in the mechanism of action on Bcl-2, a good candidate could be Nur77, which, like PTX, is able to directly convert Bcl-2 from a protector to a killer (Lin et al., 2004). This hypothesis was assessed in this study by looking at the ability of Nur77 to specifically interact with Bcl-2 in its disordered loop domain, and using experiments aimed at detecting direct competition between PTX and Nur77 for the binding to Bcl-2. Moreover, in this study we demonstrated that Nur77 is able to bind not only to Bcl-2, but also to β-tubulin, even in this resembling the behaviour of PTX. Furthermore, stable transformation with Nur77 leads to posttranscriptional changes at the Bcl-2 level similar to those detected in PTX-treated cells at the mitochondrial level and the overexpression of class III β-tubulin in the microtubule compartment. These features demonstrate that stable transfection of Nur77 is able to reproduce the same phenotypic effects observable under PTX exposure. Therefore, the authors' findings are consistent with the interpretation that PTX is a peptidomimetic compound able to mimic the pro-apoptotic activity of Nur77 (Li et al., 2000; Lin et al., 2004). This prompted them to analyze the structure of PTX, which revealed that it is able to mimic a peptide structure containing both an LXXLL/SH3/WW-like domain and a polyproline domain. On this basis, the authors designed a peptide molecular scaffold which includes the synthesized peptides PEP1 and PEP2, predicted to display the same biological activity as PTX. The activity of the designed peptides confirmed the authors' hypothesis that PTX is a peptidomimetic compound bearing the same death signal that is delivered physiologically by Nur77, namely disruption of microtubule dynamics and Bcl-2-mediated PTPC opening. In this context, the increased levels of TUBB3 in cells that stably overexpressed Nur77, suggest that TUBB3 provides a pathway for avoiding Nur77-dependent cell death. Nature has performed combinatorial chemistry for hundreds of million years. This led to the biosynthesis of PTX and the other agents sharing its peptidomimetic pharmacophore, including the viral protein of HIV-1, that are able to mimic so effectively the death message delivered by Nur77. The present studies allowed us to design a peptide scaffold containing this message which led to the synthesis of PEP1-2, thus providing a crucial tool to manipulate cell-apoptosis.

### REFERENCES

Agrawal,V. and Kishan,K.V. (2002). Protein Pept. Lett. 9, 185-193.
Andre,N., Braguer,D., Brasseur,G., Goncalves,A., Lemesle-Meunier,D., Guise,S., Jordan,M.A., and Briand,C. (2000). Cancer Res. 60, 5349-5353.
Burkhart,C.A., Kavallaris,M., and Band,H.S. (2001). Biochim. Biophys. Acta 1471, O1-O9.
Chang,B.S., Minn,A.J., Muchmore,S.W., Fesik,S.W., and Thompson,C.B. (1997). EMBO J. 16, 968-977.
Chen,D., Wang,M., Zhou,S., and Zhou,Q. (2002). EMBO J. 21, 6801-6810.
de Mareuil,J., Carre,M., Barbier,P., Campbell,G.R., Lancelot,S., Opi,S., Esquieu,D., Watkins,J.D., Prevot,C., Braguer,D., Peyrot,V., and Loret,E.P. (2005). Retrovirology. 2, 5.
Evtodienko,Y.V., Teplova,V.V., Sidash,S.S., Ichas,F., and Mazat,J.P. (1996). FEBS Lett. 393, 86-88.
Fang,G., Chang,B.S., Kim,C.N., Perkins,C., Thompson,C.B., and Bhalla,K.N. (1998). Cancer Res. 58, 3202-3208.
Fasan,R., Dias,R.L., Moehle,K., Zerbe,O., Vrijbloed,J.W., Obrecht,D., and Robinson,J.A. (2004). Angew. Chem. Int. Ed Engl. 43, 2109-2112.
Ferlini,C., Biselli,R., Nisini,R., and Fattorossi,A. (1995). Cytometry 21, 284-293.
Ferlini,C., Raspaglio,G., Mozzetti,S., Distefano,M., Filippetti,F., Martinelli,E., Ferrandina,G., Gallo,D., Ranelletti,F.O., and Scambia,G. (2003). Mol. Pharmacol. 64, 51-58.
Greenwald RB et al.,(2003). Adv Drug Deliv Rev 55(2):217-50
Haldar,S., Chintapalli,J., and Croce,C.M. (1996). Cancer Res 56, 1253-1255.
Harris JM and Chess RB. (2003). Nat Rev Drug Discov. 2(3):214-21.
Leu,J.G., Chen,B.X., Diamanduros,A.W., and Erlanger,B.F. (1994). Proc. Natl. Acad. Sci. U. S. A 91, 10690-10694.
Li,H., Kolluri,S.K., Gu,J., Dawson,M.I., Cao,X., Hobbs,P.D., Lin,B., Chen,G., Lu,J., Lin,F., Xie,Z., Fontana,J.A., Reed,J.C., and Zhang,X. (2000). Science 289, 1159-1164.
Lin,B., Kolluri,S.K., Lin,F., Liu,W., Han,Y.H., Cao,X., Dawson,M.I., Reed,J.C., and Zhang,X.K. (2004).. Cell 116, 527-540.
Lowe,J., Li,H., Downing,K.H., and Nogales,E. (2001). J Mol. Biol. 313, 1045-1057.
Mayer,B.J. (2001). J. Cell Sci. 114, 1253-1263.
Ojima,I., Chakravarty,S., Inoue,T., Lin,S., He,L., Horwitz,S.B., Kuduk,S.D., and Danishefsky,S.J. (1999). Proc. Natl. Acad. Sci. U. S. A 96, 4256-4261.
Pillai O and Panchagnula R. (2001). Curr Opin Chem Biol.5(4):447-51
Puntervoll, P., Linding, R., Gemund, C., Chabanis-Davidson, S., Mattingsdal, M., Cameron, S., Martin, D.M., Ausiello, G., Brannetti, B., Costantini, A., Ferre, F., Maselli, V., Via, A., Cesareni, G., Diella, F., Superti-Furga, G., Wyrwicz, L., Ramu, C., McGuigan, C., Gudavalli, R., Letunic, I., Bork, P., Rychlewski, L., Kuster, B., Helmer-Citterich, M., Hunter, W.N., Aasland, R., and Gibson, T.J. (2003). Nucleic Acids Res. 31(13):3625-30.
Rodi,D.J., Janes,R.W., Sanganee,H.J., Holton,R.A., Wallace,B.A., and Makowski,L. (1999). J Mol Biol 285, 197-203.
Voehringer,D.W. (1999). Free Radic. Biol Med 27, 945-950.
Wu,J.H., Batist,G., and Zamir,L.O. (2000). Anticancer Drug Des 15, 441-446.

## Claims

1. A peptide consisting of the amino acid sequence LVPPPPIVGYF or LVPPPPIVPYF.

2. A fused chimeric protein comprising the peptide according to claim 1.

3. A conjugated protein comprising the peptide according to claim 1.

4. The peptide according to claim 1 for medical use.

5. The peptide according to claim 1 for use as an anti-cancer medicament.

6. Use of the peptide according to claim 1 for the preparation of an anti-cancer medicament.

7. Pharmaceutical composition comprising a therapeutic amount of the peptide according to claim 1 and suitable diluents and/or excipients and/or adjuvants.

## Patentansprüche

1. Peptid, bestehend aus der Aminosäuresequenz LVPPPPIVGYF oder LVPPPPIVPYF.

2. Chimäres Fusionsprotein, welches das Peptid nach Anspruch 1 umfasst.

3. Konjugiertes Protein, welches das Peptid nach Anspruch 1 umfasst.

4. Peptid nach Anspruch 1 zur medizinischen Verwendung.

5. Peptid nach Anspruch 1 zur Verwendung als Antikrebsmedikament.

6. Verwendung von dem Peptid nach Anspruch 1 zur Herstellung von einem Antikrebsmedikament.

7. Pharmazeutische Zusammensetzung, die eine therapeutische Menge von dem Peptid nach Anspruch 1 und geeignete Verdünnungsmittel und/oder Hilfsstoffe und/oder Zusatzstoffe umfasst.

## Revendications

1. Peptide constitué par la séquence d'acides aminés LVPPPPIVGYF ou LVPPPPIVPYF.

2. Protéine chimérique fusionnée qui comprend le peptide selon la revendication 1.

3. Protéine conjuguée qui comprend le peptide selon la revendication 1.

4. Peptide selon la revendication 1 destiné à une utilisation médicale.

5. Peptide selon la revendication 1 destiné à être utilisé en tant que médicament anticancéreux.

6. Utilisation du peptide selon la revendication 1 pour la préparation d'un médicament anticancéreux.

7. Composition pharmaceutique comprenant une quantité thérapeutique du peptide selon la revendication 1 et des diluants et/ou excipients et/ou adjuvants adaptés.


### SEQUENCE LISTING

<110> Università Cattolica Sacro Cuore Roma CAMPIANI, Giuseppe FATTORUSSO, Caterina
<120> Novel taxane related peptides and uses thereof
<130> BE 95743
<160> 5
<170> PatentIn version 3.3
<210> 1
   <211> 12
   <212> PRT
   <213> Artificial
<220>
   <223> synthetic peptide
<220>
   <221> X
   <222> (1)..(2)
   <223> L, Y, F, V, I, W or unnatural amino acids
<220>
   <221> X
   <222> (7)..(8)
   <223> L, Y, F, V, I, W or unnatural amino acids
<220>
   <221> X
   <222> (9)..(10)
   <223> G, P, A, N or unnatural amino acids
<220>
   <221> X
   <222> (11)..(12)
   <223> L, Y, F, V, I, W or unnatural amino acids
<400> 1
   Xaa Xaa Pro Pro Pro Pro Xaa Xaa Xaa Xaa Xaa Xaa
   1               5                   10
<210> 2
   <211> 33
   <212> DNA
   <213> Artificial
<220>
   <223> synthetic forward primer
<400> 2
   ttaagcttat ggcgcacgct gggagaacag ggt 33
<210> 3
   <211> 33
   <212> DNA
   <213> Artificial
<220>
   <223> synthetic reverse primer
<400> 3
   cctctagatt cacttgtggc ccagataggc acc 33
<210> 4
   <211> 30
   <212> DNA
   <213> Artificial
<220>
   <223> synthetic forward primer
<400> 4
   ttacaggatc cagatgccct gtatccaagc 30
<210> 5
   <211> 30
   <212> DNA
   <213> Artificial
<220>
   <223> synthetic reverse primer
<400> 5
   gcccgaattc ggtcagaagg gcagcgtgtc 30